**Europäisches Patentamt**

**European Patent Office**

**Office européen des brevets**

(11) Veröffentlichungsnummer: **0 231 769**
**A2**

(12) # EUROPÄISCHE PATENTANMELDUNG

(21) Anmeldenummer: **87100288.7**

(22) Anmeldetag: **12.01.87**

(51) Int. Cl.³: **A 61 B 3/10**
**G 01 N 21/47**

(30) Priorität: **21.01.86 CH 217/86**

(43) Veröffentlichungstag der Anmeldung:
**12.08.87 Patentblatt 87/33**

(84) Benannte Vertragsstaaten:
**AT BE CH DE FR GB IT LI NL SE**

(71) Anmelder: **Interzeag AG**
**Rietbachstrasse 5**
**CH-8952 Schlieren(CH)**

(72) Erfinder: **Flammer, Josef, PD Dr. med.**
**Schülerweg 1**
**CH-3043 Üttligen(CH)**

(74) Vertreter: **Fillinger, Peter, Dr.**
**Rütistrasse 1a**
**CH-5400 Baden(CH)**

(54) Verfahren und Vorrichtung zum Messen der Trübung der Linse des menschlichen Auges.

(57) Zum Messen und Quantifizieren der Linsentrübung im menschlichen Auge wird vorgeschlagen, dass mindestens längs einem ersten Pfad (6) ein elektromagnetischer Strahl durch die Iris in die Linse des Auges gelenkt wird. Alsdann wird mit einem Detektor der längs einem zweiten Pfad reflektierte Teil des in der Linse reflektierten Strahls erfasst und die gemittelte Stärke der vom Detektor erfassten Streurate des Strahls als Mass für die Linsentrübung gemessen.

Fig.1

EP 0 231 769 A2

Verfahren und Vorrichtung zum Messen der Trübung der Linse des menschlichen Auges

Mit den seit einiger Zeit eingeführten Methoden zur einfachen Quantifizierung von perimetrisch feststellbaren Gesichtsfeldschädigungen konnte das Auftreten mehr oder weniger diffuser Schäden bei Glaukom-Erkrankungen erneut bestätigt werden. Mit den Indizes MD (Mean Defect), CLV (Corrected Loss Variance) SF (Short Term Fluctuation) wird das Gesichtsfeld zur Hauptsache beschrieben (wobei der Index MD die diffusen Schäden widergibt). Solche diffuse Schäden können nun sowohl von einem Glaukom wie von einem Katarakt verursacht werden. Es ist für den behandelnden Arzt von grosser Wichtigkeit, zu wissen, was nun die Ursache eines erhöhten "Mean Defect" ist, respektive, den Anteil eines Kataraktes von dem eines Glaukoms zu trennen.

Bisher war es nicht möglich, eine Linsentrübung zu quantifizieren. Die Ärzte beurteilten die Trübung subjektiv mit Ausdrücken wie "leichte Trübung", "mittlere Trübung" etc.

Die vorliegende Erfindung stellt sich die Aufgabe, die Linsentrübung zu quantifizieren bzw. zu messen, so dass die bisherige subjektive Beurteilung ersetzt werden kann durch eine Aussage, die den durch einen Katarakt verursachten Anteil des "Mean Defects" angibt.

Erfindungsgemäss wird diese Aufgabe gelöst durch die kennzeichnenden Merkmale der Ansprüche 1 und 6.

Der Begriff "Streurate" in der vorliegenden Beschreibung bedeutet differentieller Wirkungsquerschnitt.

Anhand der beiliegenden schematischen Zeichnung wird die Erfindung beispielsweise erläutert. Es zeigen:

Fig. 1 ein Prinzipschema zur Erläuterung des Verfahrens und
Fig. 2 ein Blockschema zur Durchführung des Verfahrens.

Vor der Netzhaut 1 des Auges und unmittelbar hinter der Iris 2
liegt die Linse 3. In der Linse 3 können krankheits- oder
altersbedingt Trübungen 4 auftreten, die die Leistungsfähigkeit des Auges auch bei intakter Netzhaut 1 beeinträchtigen.
Zur Messung der Trübung der Linse 3 wird mittels einer Lichtquelle 5 ein Lichtbündel längs einem ersten Lichtpfad 6 durch
die Blendenöffnung der Iris 2 in die Linse 3 gerichtet. Das
auf die Trübung 4 auftreffende Licht wird teilweise in diffuser
Weise gestreut. Dabei ist die Streuung umso stärker, je stärker
die Trübung ist. Das längs einem zweiten Lichtpfad 7 reflektierte Streulicht wird mittels eines Fotodetektors 8 detektiert und der Fotostrom an einem Nanoampèremeter 9 zur Anzeige
gebracht. Es ist selbstverständlich, dass in den Lichtpfaden
6 und 7 Umlenkspiegel und erforderlichenfalls auch Filter
und weitere optische Elemente angeordnet sein können. Die
Lichtquelle 5 ist vorzugsweise so gestaltet, dass sie monochromatisches Licht abgibt. Mittels eines der Lichtquelle
zugeordneten Modulators 10 kann die Lichtquelle mit
modulierter Intensität betrieben sein. Dabei kann z.B. einem als Orientierungsstrahl dienenden Lichtbündel
periodisch eine grössere Lichtstärke aufgepulst werden. Das
von der Lichtquelle ausgesandte Licht kann im Infrarot- oder
UV-Lichtbereich oder im Bereich sichtbaren Lichts liegen.
Der Winkel $\omega$ unter dem sich die beiden Lichtpfade innerhalb
der Linse 3 schneiden liegt zwischen 5° und 90°, vorzugsweise
10° und 40°.

Die Lichtquelle 5 kann die Lichtquelle einer Spaltlampe sein,
wobei mittels deren Beobachtungsoptik längs dem zweiten Lichtpfad 7 das Augeninnere durch die Irisöffnung betrachtet werden
kann. Ein Teil des längs dem zweiten Lichtpfad 7 reflektierten
Lichtes kann zwischen dem Beobachtungsobjektiv und einem der
beiden Okulare mittels eines halbdurchlässigen Spiegels auf
den Fotodetektor 8 umgelenkt werden. Es besteht indessen auch
die Möglichkeit, eines der Okulare durch einen den Fotodetektor
8 enthaltenden Lichtmesskopf zu ersetzen. Es wird vorzugsweise,
gegebenenfalls unter Vorschaltung eines entsprechenden Filters,
ein Fotodetektor 8 mit der gleichen spektralen Empfindlichkeitsverteilung wie das menschliche Auge gewählt. Damit ist
eine Quantifizierung der Linsentrübung gewährleistet, die
dem subjektiven Empfinden des Patienten entspricht.

Als Fotodetektor 8 kann auch eine Videokamera verwendet werden,
deren Bild auf einem geeigneten Medium elektronisch gespeichert
wird. Mittels eines entsprechenden Computer-Programms können die gespeicherten Werte als Kurve ausgedruckt, integriert oder sonstwie
gemittelt und in einem einzigen Mittelwert angezeigt werden.

Anstelle eines Nanoampèremeters 9 kann auch eine elektronisch gesteuerte Messwerteinrichtung verwendet werden.

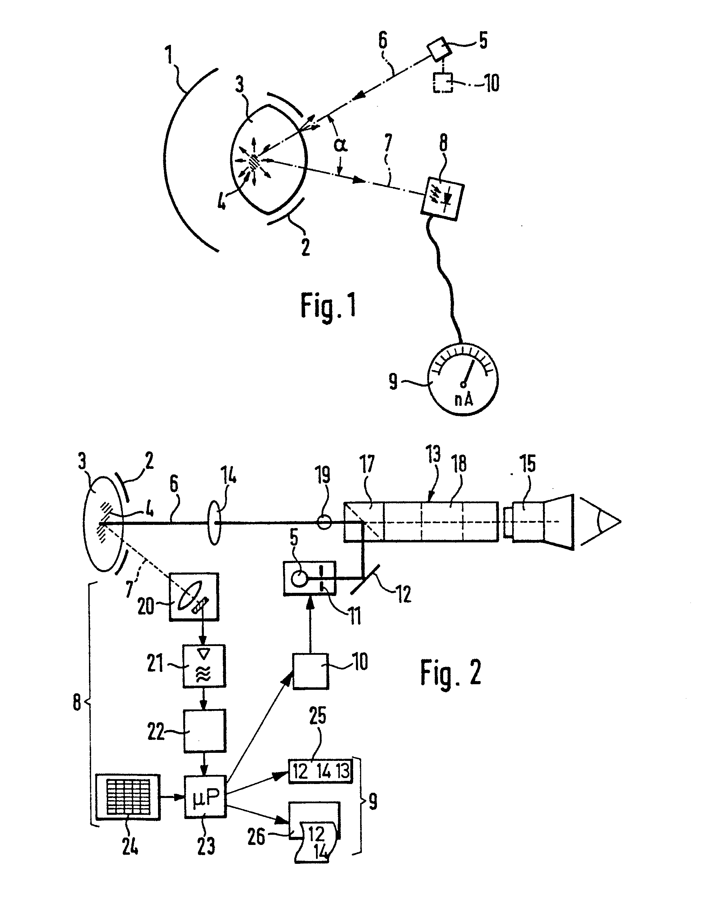

Beim Ausführungsbeispiel nach Fig. 2 bezeichnen gleiche Hinweisziffern gleiche Funktionsteile wie im ersten Ausführungsbeispiel. Die Lichtquelle 5, Erzeugerin des Messlichts, besteht aus einer Leuchtdiode im tiefroten Bereich (zirka 690
nm). Von ihr führt der erste Lichtpfad 6 durch eine veränderbare Blende 11, welche einen Strahl von vorzugsweise 1,5 oder
3 mm Durchmesser erzeugt. Die Blende kann von der Kreisform
abweichen, um einen möglichst grossen Anteil unerwünschter
Reflexion des Strahls (6) auf der Cornea des Auges zu verhindern. Mittels eines Umkehrspiegels 12 wird der Lichtstrahl
in eine Optik 13 geleitet, bei der links das Hauptobjektiv
14 angedeutet ist. Dieses dient einerseits der Abbildung der
Iris 2 im Okkular 15 des Beobachtungsfernrohrs und anderseits
der Abbildung des auf dem ersten Lichtpfad 6 ins Patientenauge
geführten roten Sendelichtstrahls. Die Optik 13 weist weiter
einen halbdurchlässigen Strahlteilerwürfel 17 auf, der die
optischen Strahlengänge des ersten Lichtpfades 6 und des
Beobachtungsfernrohres mischt. Zwischen dem Strahlenteilerwürfel 17 und dem Okkular 15 ist im Beobachtungsfernrohr ein
Abbé-Prisma 18 zur Bildumkehr angeordnet. In den ersten Lichtpfad 6 wird zudem ein Fixationspunkt 19 für die Blickrichtung
des Patienten eingeblendet.

Der aus dem Katarakt 4 (Linsentrübung) auf dem zweiten Lichtpfad 7 reflektierte Anteil des modulierten, roten Streulichts
wird im Fotodetektor 8 zuerst auf einen mit Optik und Vorverstärker ausgerüsteten Fotoempfänger 20 geleitet und dort gemessen. In einem anschliessenden Verstärker und Bandpassfilter 21 werden die Störeinflüsse reduziert und das Signal auf ein normal verarbeitbares Niveau angehoben. Im anschliessenden Demodulator 22 wird der verstärkte und gefilterte Streulichtpegel demoduliert und von Rauschanteilen befreit. Dadurch werden die mitempfangenen Komponenten des Umgebungslichtes (Tages- und Kunstlicht) wieder vom modulierten Leuchtdiodenlicht (Nutzsignal) getrennt. Das demodulierte Nutzsignal wird über einen Analog-/Digitalwandler einem Mikroprozessor 23 mit einer Eingabetastatur 24 zugeleitet, dort mittels geeigneter mathematischer Algorithmen weiter verarbeitet und in einem alphanumerischen Display 25 zur Anzeige gebracht. Eine graphische Darstellung mit einem Printer 26 ist ebenfalls möglich. Der Mikroprozessor 23 steuert weiter den der Lichtquelle 5 zugeordneten Modulator.

Zusätzlich (oder anstelle) der Stärke des aus der Augenlinse längs des zweiten Pfades 7 reflektierten Lichts kann auch dessen Polarisation im Katarakt 4 gemessen werden, wenn die Lichtquelle 5 unpolarisiertes Licht aussendet.

Wird von der längs dem zweiten Pfad 7 reflektierten elektromagnetischen Strahlung mit dem Detektor 8 das elektrische oder magnetische Feld erfühlt, kann die Energiedichte gemessen werden, welche direkt proportional der Streurate ist.

Sendet die Lichtquelle 5 monochromatisches Licht, so kann nach dem Prinzip der Fresnel'schen Interferenzversuche gemessen werden, wie weit durch die Streuung die Monochromatie des Lichts zerstört wird (inelastische Streuung), was ein Mass für die Streurate darstellt.

Patentansprüche

1. Verfahren zum Messen der Trübung der Linse eines menschlichen Auges, dadurch gekennzeichnet, dass mindestens längs einem ersten Pfad (6) ein Strahl elektromagnetischer Wellen durch die Iris in die Linse des Auges gelenkt wird, dass mit einem Detektor der längs einem zweiten Pfad (7) der in der Linse durch Streuung reflektierte Anteil des Strahls erfasst und die gemittelte Stärke der vom Detektor erfassten Streurate des Strahls als Mass für die Linsentrübung gemessen wird.

2. Verfahren nach Anspruch 1, dadurch gekennzeichnet, dass der Strahl elektromagnetischer Wellen ein Lichtstrahl ist.

3. Verfahren nach Anspruch 2, dadurch gekennzeichnet, dass der Lichtstrahl monochromatisches und/oder kohärentes und/oder polarisiertes Licht ist.

4. Verfahren nach einem der Ansprüche 1 bis 3, dadurch gekennzeichnet, dass der elektromagnetische Strahl pulsierend und/oder modulierend verändert wird.

5. Verfahren nach einem der Ansprüche 1 bis 4, dadurch gekennzeichnet, dass der zweite Pfad (7) vom ersten (6) unter einem konstanten Winkel von 0 bis 90°, vorzugsweise 10 bis 40°, wegführt.

0231769

6. Vorrichtung zur Durchführung des Verfahrens nach Anspruch 1, gekennzeichnet durch wenigstens eine elektromagnetische Strahlenquelle (5), durch wenigstens einen daran anschliessenden auf ein Ziel (3) einrichtbaren Strahlenpfad (6), durch mindestens einen zweiten, vom Ziel (3) wegführenden Strahlenpfad (7), der mit dem ersten (6) im Bereich des Ziels (3) einen Winkel einschliesst, und durch eine an den zweiten Strahlenpfad (7) angekoppelte Messeinrichtung (8, 9) zum Messen der Streurate des im Zielbereich durch Streuung reflektierten Anteils des elektromagnetischen Strahls.

7. Vorrichtung nach Anspruch 6, dadurch gekennzeichnet, dass die Strahlenpfade (6, 7) im Zielbereich einen Winkel von 0 bis 90°, vorzugsweise 10 bis 40°, einschliessen.

8. Vorrichtung nach einem der Ansprüche 6 oder 7, dadurch gekennzeichnet, dass die Quelle (5) elektromagnetischer Strahlung eine Lichtquelle ist.

9. Vorrichtung nach einem der Ansprüche 6 bis 8, dadurch gekennzeichnet, dass der Lichtquelle (5) ein Modulator (10) zur Erzeugung modulierten und/oder pulsierenden Lichtes zugeordnet ist.

10. Vorrichtung nach einem der Ansprüche 6 bis 9, dadurch gekennzeichnet, dass eine Beobachtungsoptik (13) in den ersten Pfad (6) eingekoppelt ist.

11. Vorrichtung nach Anspruch 9, dadurch gekennzeichnet, dass sie eine Spaltlampe aufweist, und dass die Lichtquelle (5) die Lichtquelle der Spaltlampe ist, dass der zweite Pfad (7) längs der optischen Achse des Okularteils der Spaltlampe geführt ist, und dass ein Fotodetektor (8) hinter dem Objektiv der Spaltlampe in den zweiten Lichtpfad eingekoppelt ist.

12. Vorrichtung nach Anspruch 10, dadurch gekennzeichnet, dass die Beobachtungseinrichtung mit einem konventionellen Okular (15) oder mit einer elektronischen Bildaufnahme- und Wiedergabeeinheit verbunden ist.

Fig.1

Fig. 2